# EUROPEAN PATENT APPLICATION

(11) **EP 3 932 438 A1**
(43) Date of publication of application: **05.01.2022**
(21) Application number: 20183585.7
(22) Date of filing: 02.07.2020
(51) Int. Cl.: A61L 29/08, A61L 29/12

(54) **HYDROPHILIC MEDICAL DEVICE ASSEMBLY**

(71) Applicant: Dentsply IH AB, 431 21 Mölndal (SE)
(72) Inventor: ERIKSSON, Karsten, 475 45 FOTÖ (SE); SANDBERG, Michael, 436 39 ASKIM (SE); WESTLING, Åsa, 437 32 LINDOME (SE); ISELAU, Frida, 438 34 LANDVETTER (SE); DAHLBERG, Niklas, 443 38 LERUM (SE)
(74) Representative: Lind Edlund Kenamets Intellectual Property AB

(57) **Abstract**

A medical device assembly comprises a medical device, such as a urinary catheter, comprising an insertable part and a non-insertable part. The insertable part comprises an elongate shaft provided with a hydrophilic coating or surface. The assembly further comprises a wetting fluid compartment arranged to encircle at least a part of the medical device, and preferably at least a part of the non-insertable part, and an aqueous fluid arranged in the wetting fluid compartment. At least one of the aqueous fluid and the hydrophilic coating or surface comprises at least one surfactant. The hydrophilic coating or surface is maintained essentially separated from the aqueous fluid, and arranged to become wetted by movement of the elongate shaft through the wetting fluid compartment.

## Description

### Technical field of the invention

The present invention relates to a medical device assembly, such as a catheter assembly, and preferably a urinary catheter assembly. The invention is particularly related to hydrophilic catheters, and specifically to hydrophilic urinary catheters. The invention also relates to a method for wetting such a hydrophilic medical device.

### Background

The present invention relates to a medical device assembly, and in particular a urinary catheter assembly. Urinary catheters are commonly used for draining urine from the bladder. Urinary catheters can be of an indwelling type, for long term use, such as days or even weeks, or for intermittent use, whereby the catheters are used for a single draining procedure, typically lasting a few minutes. Intermittent urinary catheters are e.g. used by a large group of persons for self-catheterization, which is a daily-life procedure, taking place several times a day. Typically, catheters for intermittent catheterization are used by patients suffering from urinary retention, due to e. g. spinal cord injury, Multiple Sclerosis or Prosthatic Hyperplasia. Using an intermittent catheter, the bladder may be drained through a natural or artificial urinary canal. Many catheters, such as those for intermittent catheterization, are provided with a hydrophilic coating or the like, providing a smooth and slippery surface when wetted, for safe and comfortable insertion in the urinary canal.

Individuals who suffer from urinary incontinence will normally self-catheterize several times a day. Self-catheterization involves removing the catheter assembly from its package and inserting and advancing the catheter tube through the user's urethra. However, preparation and manipulation of known catheter assemblies is often complicated and tedious. Further, often users of intermittent urinary catheters have limited or diminished dexterity, e.g. as a result of spinal cord injuries. Also, users of intermittent catheters are often required to self-catheterize outside the privacy of the home, such as in public restrooms. Thus, for these and other reasons, it is desirable that the intermittent catheters are provided in discrete packaging that is easy to open and manipulate, which are compact and portable, and wherein the catheter can be prepared for use in a simple manner, and without any risk of inadequate wetting of the hydrophilic coating.

In particular, male catheters are relatively long, making the catheter assemblies large and difficult to handle, and requiring a relatively large amount of wetting liquid to ensure that the hydrophilic coating is adequately wetted when it is to be used.

It is known to have urinary catheters where a wetting liquid is stored in direct contact with the hydrophilic coating during storage. Hereby, the catheter will be immediately ready for use. However, in such assemblies, there is a risk that the hydrophilic coating deteriorates during the sterilization or the wetted storage. Thus, relatively complex and costly coating procedures and/or wetting liquids are necessary to alleviate this problem, and the shelf-life of such products is often limited. In addition, barrier materials surrounding the wetted parts are needed.

It is also known to provide the wetting liquid in a separate compartment within the package. Upon use, the wetting liquid is released into the package to wet the catheter. However, in such solutions, a certain wetting time is necessary, in order to ensure that the hydrophilic coating is completely wetted, making the preparation somewhat tedious.

It is also known from e.g. EP 1642610 to arrange a wetting liquid compartment in such a way that the catheter is pulled out through it. Hereby, the hydrophilic coating is wetted while the catheter is expelled. However, with such known solutions, it is extremely difficult to ensure that the hydrophilic coating is adequately wetted. In order to be sufficiently wetted, the catheter here needs to be expelled very slowly, which makes the process complicated and cumbersome. There is also a risk that the wetting liquid will pour out from the wetting liquid container during the procedure, which will then make wetting of the remaining catheter length difficult or even impossible. A similar arrangement is disclosed in WO 19/123003. However, this solution has similar drawbacks, and also requires a relatively complex handling of the catheter assembly in order to accomplish wetting.

Thus, there is still a need for improved urinary catheter assemblies, and in particular male urinary catheter assemblies. The assembly should preferably be relatively simple and cost-efficient to produce. Further, the assembly should be easy and quick to prepare, and allow intuitive handling for the user, and at the same time ensure that the hydrophilic coating or surface becomes adequately wetted in a safe and secure way, which is not dependent on the user's skill etc. The assembly should also preferably be rather small and light, so that it can easily be carried around by the user in his/her daily life.

Similar needs for improvement are also present in many other types of hydrophilic medical devices.

### Summary of the invention

It is therefore an object of the present invention to provide a medical device assembly which at least alleviates the above-discussed problems.

This object is obtained by means of a medical device assembly, and a corresponding method, in accordance with the appended claims.

According to a first aspect of the present invention, there is provided a medical device assembly comprising:
a medical device comprising an insertable part and a non-insertable part, wherein the insertable part comprises an elongate shaft provided with a hydrophilic coating or surface;
a wetting fluid compartment arranged to encircle at least a part of the medical device, and preferably at least a part of the non-insertable part; and
an aqueous fluid arranged in the wetting fluid compartment, wherein at least one of the aqueous fluid and the hydrophilic coating or surface comprises at least one surfactant;
wherein the hydrophilic coating or surface is maintained essentially separated from the aqueous fluid, and arranged to become wetted by movement of the elongate shaft through the wetting fluid compartment.

By "surfactant" is in the present application meant all molecules which have surface active properties, and which can decrease the surface tension or improve wetting by a fluid on a substrate.

In the present invention, one or more surfactants is present in either the wetting fluid or the hydrophilic coating/surface, or in both the wetting fluid and the hydrophilic coating/surface. If surfactant(s) are provided in both the wetting fluid and in the hydrophilic coating/surface, the surfactant(s) may be the same surfactant(s), or different surfactants.

The present invention is based on the surprising realization that a wetting arrangement as the ones disclosed in the above-discussed EP 1642610 and WO 19/123003 (said documents hereby being incorporated by reference in their entirety) can be greatly improved by the use of at least one surfactant in the aqueous fluid and/or in the hydrophilic coating/surface. It has surprisingly been found by the present inventors that complete and adequate wetting can thereby be obtained with such types of arrangements in a very short period of time. Hereby, the catheter can be pulled out at essentially any desired speed, and for example be completely released from the wetting fluid compartment in a second or less, and still be adequately wetted.

Since the wetting fluid is in contact with only a very limited part of the elongate shaft at each time, a very limited amount of wetting fluid is needed to complete the wetting. This makes the overall medical device assembly smaller and lighter.

Further, wetting process here becomes very natural and intuitive, since wetting occurs automatically by the release of the medical device from the package and/or the wetting fluid compartment. Thus, the wetting process becomes simple, also for users having poor or diminished dexterity. There is also no risk of inadvertently forgetting to wet the medical device prior to use, or to wet the hydrophilic coating/surface insufficiently. The product hereby becomes very safe and secure for the user.

Since the hydrophilic coating/surface is maintained in a dry state during storage, it is not deteriorated over time in the same way as for pre-wetted hydrophilic coatings/surfaces. Thus, a very long shelf-life can be obtained. The hydrophilic coating/surface is hereby also not as sensitive for different types of sterilization, such as radiation sterilization, which improves the longevity and properties of the hydrophilic coating/surface. It also makes it possible to use less complex and costly hydrophilic coatings/surfaces, wetting fluids, packing materials, sterilization methods, and the like, thereby overall making the product very cost-effective.

At the same time, the medical device is here essentially as ready-to-use as if it had been pre-wetted, since the medical device would anyway need to be extracted from its package, and in the present solution, a complete wetting will be obtained automatically over the course of such a normal extraction process.

In a preferred embodiment, the aqueous fluid includes the at least one surfactant. However, additionally or alternatively, the at least one surfactant may be included in the hydrophilic coating or surface.

The critical micelle concentration (CMC) is defined as the concentration of surfactants above which micelles form and all additional surfactants added to the system go to micelles. Before reaching the CMC, the surface tension changes strongly with the concentration of the surfactant. After reaching the CMC, the surface tension remains relatively constant or changes with a lower slope. Thus, by using a concentration similar to the CMC, or above the CMC, a very controllable and predictable wetting can be obtained.

The value of the CMC for a given surfactant in a given medium depends on temperature, pressure, and on the presence and concentration of other surface active substances and electrolytes. However, in the present wetting liquid, the temperature will normally be ordinary room temperature, both during storage and use of the medical device assembly, and the pressure will be about the same as the atmospheric pressure, i.e. about 100 kPa. Further, there is preferably no other active substances and electrolytes in the aqueous liquid. Accordingly, the CMC for the surfactants in the present wetting liquid is relatively constant.

In case more than one surfactant is used, the CMC corresponds to the combined CMC for the two or more surfactants when used together.

For example, the value of CMC for sodium dodecyl sulfate in water, with no other additives or salts, at 25 °C and atmospheric pressure, is 8x10⁻³ mol/L.

Most surfactants are effective in improving wetting of a hydrophilic surface coating/surface when provided generally at CMC concentration. However, at which concentration the surfactant has best effect on the wetting of the hydrophilic coating/surface depends on the properties of each specific surfactant, and is not necessarily directly related to the CMC of that surfactant.

The total concentration of surfactant(s) may be in the range of 0.001-1 wt%, of the liquid phase (the wetting fluid or the fluid in the wetted coating/surface) and preferably in the range 0.005-0.5 wt%, and more preferably in the range 0.01-0.1 wt%, and most preferably in the range 0.01-0.08 wt%.

Expressed in Molar (M), the total concentration of surfactant(s) may be in the range of 0.001-1 mM, of the liquid phase (the wetting fluid or the fluid in the wetted coating/surface) and preferably in the range 0.002-0.7 mM, and more preferably in the range 0.003-0.6 mM.

The surfactant(s) are preferably organic compound(s), and are amphiphilic, which means that they contain both hydrophobic groups (their tails), usually a long alkyl chain, attached to hydrophilic or water solubility enhancing functional groups (their heads). Therefore, the surfactant(s) contains both a water-insoluble (or oil-soluble) component and a water-soluble component. Surfactants will diffuse in water and adsorb at interfaces between air and water or at the interface between oil and water, in the case where water is mixed with oil.

The surfactant(s) lower the surface tension (or interfacial tension) between two liquids, between a gas and a liquid, or between a liquid and a solid.

The surfactant is preferably water soluble.

Further, the surfactant is preferably non-ionic. Hereby, the interaction with human tissue is limited, and the surfactant will also not interact with e.g. ionic substances, pH buffers and similar optional additives. However, other surfactants may also be used, such as zwitterionic, anionic or cationic. Further, the surfactant preferably has relatively large chains/molecules, which further reduces any potential interaction with human tissue.

Examples of ionic surfactants that may be used in the wetting liquid and/or the hydrophilic coating/surface include one or more of alkyl sulphates (such as sodium dodecylsulphates), sodium cholate, bis(2-ethylhexyl)sulphosuccinate sodium salt, quaternary ammonium compounds, such as cetyltrimethylammonium bromide or chloride, lauryldimethylamine oxide, N- lauroylsarcosine sodium salt and sodium deoxycholate.

Examples of non-ionic surfactants that may be used in the wetting fluid and/or the hydrophilic coating/surface include one or more of alkylpolyglucosides, branched secondary alcohol ethoxylates, ethylene oxide / propylene oxide copolymers, nonylphenol ethoxylates, octylphenol ethoxylates, and specialty alkoxylates, such as Tween 80 and Tween 20.

Other examples of surfactants that may be used in the wetting fluid and/or the hydrophilic coating/surface include, but are not limited to, one or more of saponified coconut oil, vitamin E, polyoxyethylene sorbitan, monolaurate, sodium dodecyl sulfate, polysorbate, L-a- phosphatidylcholine, lecithin, stearyl stearate, sodium stearate, sodium laurate, sodium myristate, sodium myristate, sodium palmitate, sodium oleate, polyethylene glycol monododecyl ether, glycolic acid ethoxylate lauryl ether, glycolic acid ethoxylate oleyl ether, ethylene glycol monododecyl ether, polyoxyethylene glycerol ester, polyglyceryl esters, diglyceryl diisostearate, diglyceryl monolaurate, diglyceryl monooleate, docusate sodium, dioctyl sulfosuccinate sodium salt, dioctyl sodium sulfosuccinate, sodium dodecylbenzenesulfonate, perfluorobutane sulfonic acid, 3-sulfopropyl ethoxylate lau-rylphenly ether, lauric acid sodium salt, N-acylsarcosine sodium salt, and N-lauroylsarcosine sodium salt, and different types of cellulose derivatives, such as hydroxypropyl cellulose and hydroxypropyl methyl cellulose.

In preferred embodiments, the surfactant is one or more of Tween 80, Brij 35, all commercially available from Sigma Aldrich, TEGO Betain F50, commercially available from Evonik Industries, Kolliphor P188, Kolliphor P407, both commercially available from BASF, and HPMC 4M, commercially available from DuPont/Dow Chemicals.

The aqueous fluid may further include a polyol, such as glycerol, and/or low molecular weight hydrophilic polymer, such as PVP. In one embodiment, the aqueous fluid includes a relative amount of polyol or low molecular weight hydrophilic polymer in the range of 0.01-20 wt%, and preferably 0.05-15 wt%, and more preferably 0.1-10 wt%, and most preferably 1-8 wt%, such as about 5 wt%. The polyol and low molecular weight hydrophilic polymer provide protection for the wetting fluid, and in particular preserves the function of the surfactants, during radiation sterilization. The polyol and low molecular weight hydrophilic polymer reduce the crosslinking/reaction of the surfactant which may otherwise occur during radiation sterilization.

However, the polyol and low molecular weight hydrophilic polymer are optional, and in many instances, the aqueous fluid may not contain any polyol or low molecular weight hydrophilic polymer. For example, for many surfactants and concentrations, the deterioration occurring during radiation sterilization is negligible, or at least acceptable, even when there is no polyol/hydrophilic polymer in the aqueous fluid. Also, there may be no need for polyol/hydrophilic polymer in the aqueous fluid when other sterilization methods are used, such as sterilization by heat, steam, gas or the like.

The polyol is preferably a low molecular weight polyol. Preferably, the low molecular weight polyol has a molecular weight below 200 g/mol.

The low molecular weight polyol is preferably selected from the list of glycerol and low-molecular weight glycols, preferably with molecular weight lower than 200 g/mol, such as ethylene glycol, diethylene glycol, triethylenglycol, propylene glycol, dipropylene glycol, 1,3-propanediol, 1,4-butanediol, and 1,5-pentanediol.

The low molecular weight polyol is preferably glycerol.

The aqueous fluid may further comprise a pH buffer, to ensure that the pH of the fluid is maintained in a preferred range, such as in the range of 4-8. The pH buffer may be citric acid, but other buffers may also be used, such as acetic acid, phosphate buffer, carboxylic acids, amino acids, aminosulphonic acids and inorganic acids.

The aqueous fluid preferably comprises at least 80 wt% water, and more preferably at least 90 wt%, and most preferably at least 95 wt%.

In embodiments where the aqueous fluid includes a low molecular weight polyol/hydrophilic polymer, the fluid preferably comprises 80-99 wt% water and 1-10 wt% polyol/hydrophilic polymer.

The fluid is preferably a liquid. Preferably, the fluid contains primarily water, and optionally a polyol, such as glycerol, and/or a low molecular weight hydrophilic polymer. The total concentration of additives in addition to water and polyol/hydrophilic polymer, such as the surfactant, preferably is equal to or less than 5 wt%, and more preferably less than 3 wt%, and even more preferably less than 1 wt%, and most preferably less than 0.5 wt%. Apart from the surfactant, such additional additives may include one or more of an osmolality increasing agent, a pH buffer, an antibacterial agent,
fragrances, a pharmaceutical active substance, or the like.

The hydrophilic coating/surface preferably comprises polyvinylpyrrolidone (PVP).

The hydrophilic coating/surface may be arranged as a hydrophilic coating arranged on a substrate of the medical device, such as a catheter, as is per se well known in the art. However, the hydrophilic coating/surface may alternatively be arranged as an integrated part of the medical device, such as an integrated layer, or alternatively, the entire medical device, or part(s) of the medical device, may be made of a hydrophilic material. The hydrophilic coating/surface is preferably arranged to provide low friction when wetted.

In an embodiment of the invention the medical device is a catheter, and the elongate shaft is a catheter shaft arranged to be inserted into a body cavity or body passageway, and the shaft presents the hydrophilic coating/surface on an exterior surface thereof. The hydrophilic coating/surface may be a surface provided with a hydrophilic coating, for example made in accordance with EP 0 093 093 and EP 0 217 771, said documents hereby being incorporated by reference in their entirety.

Even though PVP is the preferred hydrophilic material, other hydrophilic materials may be used, such as hydrophilic polymers selected from polyvinyl compounds, polysaccharides, polyurethanes, polyacrylates or copolymers of vinyl compounds and acrylates or anhydrides, especially polyethyleneoxide, polyvinylpyrrolidone, heparin, dextran, xanthan gum, polyvinyl alcohol, hydroxy propyl cellulose, hydroxy propyl methyl cellulose, methyl cellulose, copolymer of vinylpyrrolidone and hydroxy ethylmethyl acrylate or copolymer of polymethylvinyl ether and maleinic acid anyhydride. Further, hydrophilic coating/surface is to be understood in a broad sense, and in embodiments the hydrophilic coating/surface may comprise an entire insertable section of the medical device/catheter formed of a hydrophilic material.

In preferred embodiments, the medical device is a catheter, and preferably a urinary catheter. Most preferably the medical device is a urinary catheter for intermittent, short time use. The term "short term use" indicates a use that is limited in time, and in particular limited to a time period of less than 15 minutes, and preferably less than 10 minutes, and most preferably less than 5 minutes.

The present invention is of particular advantage for male urinary catheters, i.e. urinary catheters intended for use by male users. Male catheters are relatively long. A male catheter may have a total length within the range 35-40 cm, and an insertable length of 20-35 cm. Ensuring adequate wetting of such a long insertable length is often problematic, and conventionally requires relatively large amounts of wetting fluid. However, by means of the present solution, complete and adequate wetting with a very limited amount of wetting fluid can be obtained, regardless of the length of the insertable part.

In the present application, the term "proximal" is used to indicate the end or portion of a medical device, such as a catheter, that is inserted into the body of the user, i.e. the end or portion of the medical device that during use is closer in proximity to the user's body and/or initially enters the user's body upon insertion. The term "distal" is used to refer to an end or portion of the medical device that is opposite the proximal end or portion and is typically further away from the user's body. For the sake of consistency, when the terms "distal" and "proximal" are used in the context of other components which are not intended for introduction into the user's body, "proximal" refers to the end or portion that is closer to the proximal end of the medical device when the medical device is housed within the assembly, while "distal" refers to an end or portion located opposite to such proximal end or portion.

The medical device assembly may further comprise a package accommodating the wetting fluid compartment and at least the insertable part of the medical device. In one embodiment, the package entirely encloses the medical device. However, it is also possible to have the non-insertable part wholly or partly arranged outside the package. In such an embodiment, the exposed part of the medical device, residing outside the package, may form a closure of the package.

The package may be of a flexible material, such as foil material. However, the package may also be of a more rigid material, such as rigid plastic material.

The package may, in a closed position, provide a sterile and moisture proof compartment for the insertable section of the medical device.

In one line of embodiments, the wetting fluid compartment is fixedly arranged in the package, wherein extraction of the medical device from the package simultaneously provides a movement of the elongate shaft through the wetting fluid compartment, thereby wetting the hydrophilic coating/surface. In such an embodiment, the package is opened, and the medical device is expelled, as in conventional assemblies. As the medical device is expelled, the elongate shaft passes through the wetting fluid compartment, and is consequently completely wetted as soon as it has been removed from the package in a simple one-step procedure.

In an alternative line of embodiments, the wetting fluid compartment may be releasable from the package. In such an embodiment, the medical device may first be expelled from the package together with the wetting fluid compartment, and then activated and wetted at a later time, by pulling the medical device out through the wetting fluid compartment. Such a two-step procedure is somewhat more complicated than the above-discussed one-step procedure. On the other hand, it provides the user with more control and flexibility of where and when to perform the steps.

As already discussed, the medical device may be a catheter, and preferably a urinary catheter. The catheter may have a connector, funnel shaped end or flared end arranged at the non-insertable part, such as a flared connector. The catheter preferably has an internal lumen, extending from one or more drainage openings, so-called eyes or eyelets, arranged at or in the vicinity of the proximal insertion end, to a discharge outlet arranged at or in the vicinity of the distal end. A drainage opening may be arranged at the proximal tip of the elongate shaft, i.e. forming a centrally arranged opening in the longitudinal extension of the internal lumen. Additionally, or alternatively, one or more drainage openings may be formed in the sidewall of the elongate shaft, to debouch in a direction radially outwards. In one embodiment, the elongate shaft has a rounded, closed proximal end, and one or more drainage openings arranged in the sidewall of the elongate shaft.

A part of, or the whole, catheter shaft may form an insertable part or insertable length of the catheter. At least the insertable part may be provided with the hydrophilic coating/surface, or in other ways been provided with a hydrophilic coating/surface, which exhibits a lowered friction when wetted. Further, the distal part may, at least on a part thereof, have larger cross-sectional dimensions than the catheter shaft. The distal part may e.g. be flared or funnel shaped, increasing in dimension towards the distal end, thereby enabling connection of a tube, collection bag or the like.

In one embodiment, the catheter comprises at least one drainage opening arranged in a sidewall of the elongate shaft, wherein the wetting fluid compartment has a height in a longitudinal direction of the catheter exceeding a length of the drainage opening in the longitudinal direction of the catheter. In another embodiment, the catheter comprises at least two drainage openings arranged in a sidewall of the elongate shaft, wherein the wetting fluid compartment has a height in a longitudinal direction of the catheter exceeding a length between the ends of the drainage openings being farthest apart in the longitudinal direction of the catheter. Hereby, it is ensured that the wetting fluid will be maintained in the wetting fluid compartment when the drainage openings are pulled out through the wetting fluid compartment, and not pour out through the drainage openings. By this arrangement, the drainage opening(s) will not begin to move out from the wetting fluid compartment until the drainage opening(s) have fully entered into the wetting fluid compartment, and the capillary forces will thereby maintain the wetting fluid in the wetting fluid compartment.

The wetting fluid compartment is arranged to encircle at least a part of the non-insertable part of the medical device. For example, in case of a catheter, the wetting fluid compartment may be arranged to encircle a part of the catheter shaft not provided with a hydrophilic coating/surface, or a part provided with a hydrophilic coating/surface, but which is not intended for insertion. The wetting fluid compartment may also encircle a proximal part of a flared connector, or other part connected to the catheter shaft. In one embodiment, a proximal end of the wetting fluid compartment encircles a distal part of the catheter shaft and distal end of the wetting fluid compartment encircles a proximal end of the connector.

The wetting fluid compartment may thus have a first, proximal opening and a second, distal opening. The openings are preferably closed by the non-insertable part of the medical device, ensuring that the wetting fluid does not escape from the wetting fluid compartment prior to withdrawal of the medical device through the wetting fluid compartment. Such closing can e.g. be obtained by a friction fit, whereby the inner diameters of the openings are essentially the same as the corresponding outer diameters of the non-insertable part at the positions where the openings are arranged.

In one embodiment, at least one breakable seal is further provided between the wetting fluid compartment and the elongate shaft, and preferably two breakable seals, provided on opposite sides of the wetting fluid compartment. Thus, the seals are hereby preferably arranged between the inner surface of the openings and the corresponding outer surfaces of the non-insertable part. Such seals may e.g. be formed by an adhesive, rubber or the like.

Regardless of whether seals are used or not, the diameter of the proximal opening of the wetting fluid compartment is preferably essentially the same as the outer diameter of the elongate shaft, or only slightly greater.

Usually, urinary catheters are from size 8 Ch to size 18 Ch. Charriere (Ch) is a standard gauge for catheters approximately corresponding to the outer circumference in mm. More precisely, the outer diameter of the catheter in mm corresponds to Ch divided by 3. Thus 8 Ch corresponds to a catheter with an outer diameter of 2.7 mm and 18 Ch corresponds to a catheter with an outer diameter of 6mm.

In one embodiment, the diameter of the proximal opening of the wetting fluid compartment is 0-5% greater than the outer diameter of the elongate shaft, and preferably 0.01-2% greater, and most preferably 0.1-1 % greater.

In one embodiment the diameter of the proximal opening of the wetting fluid compartment is 0-1 mm greater than the outer diameter of the elongate shaft, and preferably 0.001-0.5 mm greater, and most preferably 0.01-0.1 mm greater.

At least the proximal opening is preferably circular, and preferably both the proximal and the distal openings are circular.

In an embodiment, the distal opening of the wetting fluid compartment has a greater diameter than the diameter of the proximal opening. Hereby, a slight distance may be formed between the elongate shaft and the distal opening when the elongate shaft is pulled out through the wetting fluid compartment. This is of advantage, since it allows the hydrophilic coating/surface to swell, and still be expelled without any risk of damaging the hydrophilic coating/surface. It also ensures that some fluid will be maintained on the surface of the hydrophilic coating/surface, and be pulled along with the elongate shaft as it is pulled out from the wetting fluid compartment. This fluid on the surface will then continue the wetting and activation of the hydrophilic coating/surface also after release from the wetting fluid compartment.

Upon use, the medical device will be pulled in relation to the wetting fluid compartment, thereby breaking the seals, if such seals are present. However, since at least the proximal opening of the wetting fluid compartment is still essentially closed by the elongate shaft, the aqueous fluid will not pour out from the wetting fluid compartment during the withdrawal of the elongate shaft through the wetting fluid compartment. Due to capillary forces, the wetting fluid will instead be maintained in the compartment until the proximal tip of the elongate shaft has reached and passed through the proximal opening of the wetting fluid compartment. Any fluid remaining in the wetting fluid compartment will pour out only after complete removal of the elongate shaft, thus ensuring a complete wetting of the hydrophilic coating/surface.

Even if a small amount of fluid may accidentally pour out of the compartment during withdrawal, due e.g. to tolerances during manufacturing, this would still not be of any problem, and provided there is sufficient fluid left in the compartment this has no negative impact on the wetting of the coating. Thus, in cases where tolerances are difficult to control during manufacturing, a small surplus of wetting fluid may be used to ensure adequate wetting.

In the present solution, it is possible to use a very small volume of the aqueous fluid, since it is continuously applied to the hydrophilic coating/surface only where it is needed. Thus, in principle it suffices with a volume corresponding to the volume which is absorbed by the hydrophilic coating/surface for swelling of the same.

In one embodiment, the wetting fluid compartment may comprise an amount of the aqueous fluid in the range of 0.5-5 ml, and preferably in the range of 1-4 ml. Such a small amount of wetting fluid will be sufficient for complete wetting of a hydrophilic coating/surface of a male urinary catheter.

Due to the small amount of wetting fluid needed to complete a full and complete wetting of the hydrophilic coating/surface, a very small amount of wetting fluid will remain in the wetting fluid compartment when the elongate shaft has been completely expelled. This is of advantage, since it reduces the problem of handling a package containing loose fluid, and also reduce the risk of inadvertent spillage.

The wetting fluid compartment is preferably impermeable to the wetting fluid, and preferably made of a gas impermeable material. This ensures that moisture does not penetrate out from the wetting fluid compartment during storage, and enhances the shelf-life of the product.

According to another aspect of the present invention, there is provided a method for wetting a hydrophilic medical device comprising:
providing a medical device comprising an insertable part and a non-insertable part, wherein the insertable part comprises an elongate shaft provided with a hydrophilic coating or surface;
providing a wetting fluid compartment containing an aqueous fluid around at least a part of the non-insertable part, wherein at least one of the aqueous fluid and the hydrophilic coating or surface comprises at least one surfactant;
moving the elongate shaft in relation to the wetting fluid compartment, thereby wetting the hydrophilic coating or surface.

Hereby, similar advantages and preferred features as discussed in relation to first aspect can be obtained and used.

The moving of the elongate shaft in relation to the wetting fluid compartment can be made with such speed that the elongate shaft is moved out from the wetting fluid compartment within a time period in the range of 0.5-5 seconds, and preferably 0.5-2 seconds, and most preferably 0.7-1.5 seconds. Thus, the elongate shaft can essentially be pulled out as fast as is practically feasible.

It has surprisingly been found that wetting of the hydrophilic coating/surface is better when the catheter is pulled out fast than if pulled out at a lower speed. It is assumed that this is due to less wetting fluid being pulled up along with the shaft as it is pulled up. It is expected that most users will pull out the catheter as fast as possible, unless instructed otherwise. However, if it is expected that a lower pulling speed will be used, depending e.g. on the application and type of product, this can be compensated by providing a somewhat greater height of the wetting fluid level in the wetting fluid compartment.

The height of the level of wetting fluid within the wetting liquid compartment is preferably at least 2 cm, and more preferably at least 4 cm, and most preferably at least 6 cm.

These and other aspects of the invention will be apparent from and elucidated with reference to the embodiments described hereinafter.

### Brief description of the drawings

For exemplifying purposes, the invention will be described in closer detail in the following with reference to embodiments thereof illustrated in the attached drawings, wherein:
Fig. 1 is a top view of a urinary catheter assembly in accordance with one embodiment of the present invention;
Figs. 2a-d are cross-sectional views of the urinary catheter assembly of Fig. 1, showing various stages of an extraction and wetting process;
Fig. 3 is a top view of a urinary catheter assembly in accordance with another embodiment of the present invention; and
Figs. 4a-e are cross-sectional view of the urinary catheter assembly of Fig. 3, showing various stages in an extraction and wetting process.

### Detailed description of preferred embodiments

In the following detailed description preferred embodiments of the invention embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length of the medical device, etc. Still further, even if the detailed discussion in the following relates to a urinary catheter assembly, it is to be acknowledged that the same principles may be used for assemblies also for other hydrophilic catheters, as well as for assemblies for other hydrophilic medical devices.

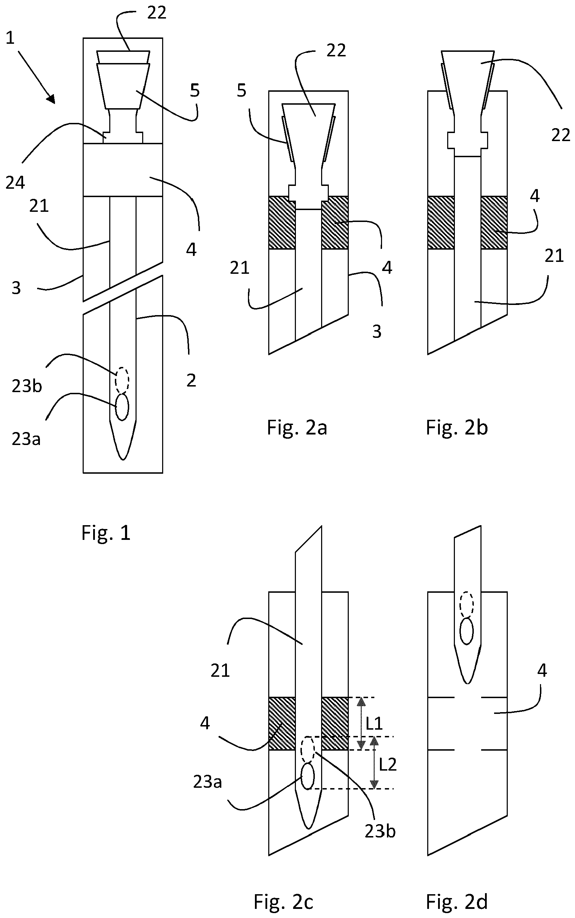

A urinary catheter assembly 1 as illustrated in Figs. 1 and 2a-d comprises a catheter 2 having an insertable section 21, comprising an insertable, proximal part, with an insertion tip, on a catheter shaft, and a non-insertable section 22, a distal part, forming a connector part. The non-insertable section 22 preferably has a larger diameter than the insertable section 21 at least on a part thereof. The rear end of the non-insertable section may be flared or funnel-shaped, and may be arranged to be connected to a tapered connection part of a urine collection bag or the like. However, the non-insertable section may alternatively have a relatively uniform cross-sectional area.

At least a part of the insertable section 21 forms an elongate shaft with an insertable length to be inserted into a natural or artificial body opening of a user, such as into a urethra of the user.

The insertable section comprises an insertion tip, which may be a closed, rounded end. Further the insertable section may comprise drainage openings 23a, 23b, arranged in the vicinity of the insertion tip, so called catheter eyes or eyelets, leading into a lumen extending through the catheter, and into a discharge outlet arranged at the rearward end of the non-insertable section 22.

The insertable section may be 80-140 mm for a female user and 200-350 mm for a male user.

The insertable section 21 may comprise a hydrophilic coating/surface, and form a hydrophilic catheter, as is per se well known in the art. The hydrophilic coating/surface may be in the form of a hydrophilic coating, for example PVP, and which provides a low-friction surface when wetted with a wetting fluid. Even though PVP is the preferred hydrophilic material, other hydrophilic materials may be used, as exemplified in the foregoing.

The catheter is accommodated in a package 3, forming a closed cavity in which at least the insertable section of the catheter is arranged. In the illustrative examples, the entire catheter is arranged within the package. The cavity is preferably sterile and moisture proof. To this end, the package may e.g. be made of a fluid impermeable material. However, materials having some permeability may also be used. The package is preferably, at least in most parts, made of a relatively flexible material, such as foil. The package may be transparent, but may alternatively be opaque or semi-opaque. The package may be in the form of a tube or hose. The package is preferably arranged to narrowly surround the catheter.

The package comprises an opening, preferably arranged above or overlying the non-insertable section 22 of the catheter. The opening may be a peel opening and/or tear opening. However, other openings may also be used, such as a cap, a lid or the like. When the package has been opened, the catheter is accessible, and the user may e.g. grip the non-insertable section and pull the catheter out of the package.

To allow convenient, clean and contamination free handling of the catheter, the assembly may further comprise an insertion aid 5. The insertion aid can be used as an aid when manipulating the catheter during or after having expelled the insertable section 21 out from the package for use.

The insertion aid 5 may, as in the illustrative examples, be arranged releasably connected to the non-insertable section 22 of the catheter. During use, the user may pull the insertion aid loose from the non-insertable section, and move it to any desired position along the length of the catheter shaft. However, in case the user does not wish to use the insertion aid, the insertion aid may be maintained in its original, non-released position during the entire use. The detachable connection between the insertion aid and the non-insertable section of the catheter may be realized in various ways, such as by friction fit, welding, adhesion, etc.

The insertion aid may be cylindrical, with a generally uniform diameter along its length. However, it may also have a slightly conical, flared shape, over its entire length or along part(s) of the length.

The insertion aid is preferably made of a relatively soft and flexible material, allowing the catheter to be gripped by applying a moderate finger pressure on the insertion aid. The insertion aid may be provided with a solid wall around its full circumference. However, the insertion aid may also comprise one or several slit openings, extending along the entire length of the insertion aid, or over only part(s) of the length. For example, a slit opening may extend from the distal end of the insertion aid, and towards the proximal end, over a length of e.g. 1/3 or 1/2 of the full length of the insertion aid. The tubular insertion aid is preferably made of a flexible plastics material.

The insertion aid may be used for catheter manipulation, thereby avoiding the need to directly touch the catheter, and in particular the insertable section of the catheter. This reduces the risk of contaminating the insertable section of the catheter prior to insertion. It may also be used to manipulate the catheter during and after withdrawal, thereby avoiding the risk of the user being contaminated by the used catheter.

Additionally, or alternatively, the wetting fluid compartment may also be used as an insertion aid, allowing indirect gripping and manipulation of the catheter.

The assembly further comprises a wetting fluid compartment 4 arranged to encircle at least a part of the non-insertable part. The wetting fluid compartment 4 comprises an aqueous fluid, capable of wetting the hydrophilic coating/surface of the catheter shaft.

The insertable section 21, and the hydrophilic coating/surface thereon, is maintained separated from the aqueous fluid in the storage position, as illustrated in Figs. 1 and 2a. Upon use, the package is opened in the distal end, and as the catheter is being expelled, the hydrophilic coating/surface is pushed into and through the wetting fluid compartment 4, as illustrated in Figs. 2b and 2c. When the catheter shaft has passed out from the wetting fluid compartment, as shown in Fig. 2d, the hydrophilic coating/surface has become wetted by the aqueous fluid, and any fluid remaining in the wetting fluid compartment at that time will pour out through the proximal opening and into the package. However, since a very limited amount of fluid needs to be used, the amount of remaining fluid will be very small.

In the embodiment illustrated in Figs. 1 and 2a-d, the wetting fluid compartment 4 is fixedly arranged in the package 3, wherein extraction of the medical device from the package simultaneously provides a movement of the elongate shaft through the wetting fluid compartment, thereby wetting the hydrophilic coating/surface. In such an embodiment, the package is opened, and the medical device is expelled, as in conventional assemblies. As the medical device is expelled, the elongate shaft passes through the wetting fluid compartment, and is consequently completely wetted as soon as it has been removed from the package in a simple one-step procedure.

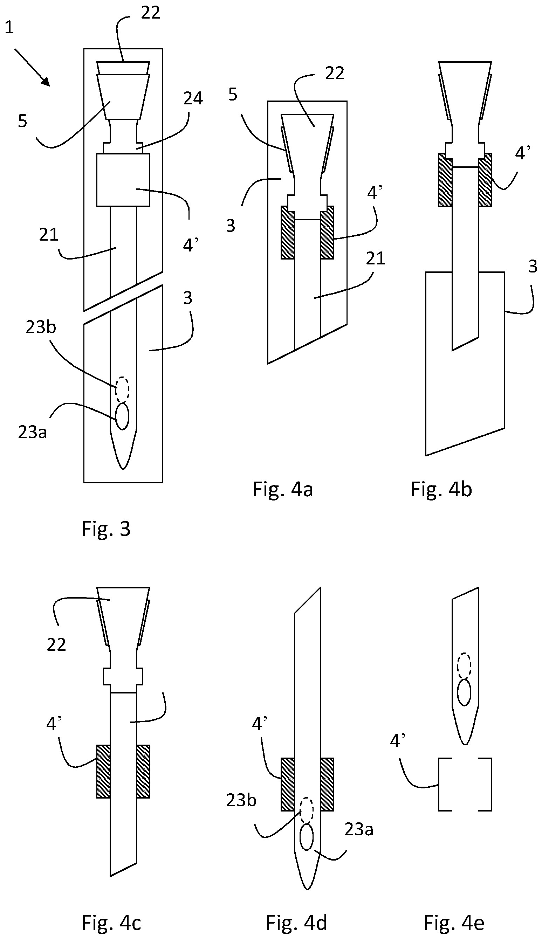

In an alternative embodiment, illustrated in Figs. 3 and 4a-e, the wetting fluid compartment may be releasable from the package. Apart from this difference, the catheter assembly may be similar or identical to the above-discussed first embodiment.

In the embodiment of Figs. 3 and 4a-e, the wetting fluid compartment 4' is attached to the non-insertable section 22 of the catheter, e.g. in the same way as in the first discussed embodiment. However, the wetting fluid compartment 4' is here not connected to the package. In the storage position, as shown in Figs. 3 and 4a, the package is closed. Upon use, the package is opened in the distal end, and the catheter is expelled out from the package, as illustrated in Fig. 4b. The wetting fluid compartment remains attached to the catheter, and is pulled out from the package along with the catheter. In a subsequent step, illustrated in Figs. 4c and 4d, the wetting fluid compartment 4' is then moved in relation to the catheter shaft, so that it moves towards the proximal end. When the catheter shaft has been completely moved through the wetting fluid compartment, any remaining fluid will pour out from the wetting fluid compartment, and e.g. into a sink or a toilet.

In the same way as in the first discussed embodiment, the hydrophilic coating/surface will be activated and wetted as the catheter shaft is moved through the wetting fluid compartment, but in this second embodiment, this will be made in a step separate from the extraction of the catheter from the package.

In both the above-discussed embodiments, the wetting fluid compartment 4, 4' may have a height in a longitudinal direction of the catheter exceeding a length of the drainage opening in the longitudinal direction of the catheter. If the catheter comprises at least two drainage openings 23a, 23b arranged in the sidewalls of the elongate shaft, as in the illustrative examples, the wetting fluid compartment 4, 4' preferably has a height in a longitudinal direction of the catheter exceeding a length between the ends of the drainage openings being farthest apart in the longitudinal direction of the catheter. This is illustrated in detail in Fig. 2c, where the height of the wetting fluid compartment is shown as L1 and the distance between the ends of the drainage openings 23a and 23b being farthest apart is L2. Thus, here L1 is preferably greater than L2.

The wetting fluid compartment is arranged to encircle at least a part of the non-insertable part of the medical device. The wetting fluid compartment may e.g. be arranged to encircle a part of the catheter shaft not provided with a hydrophilic coating/surface, or a part provided with a hydrophilic coating/surface, but which is not intended for insertion. The wetting fluid compartment may also encircle a proximal part of a flared connector, or other part connected to the catheter shaft. In one embodiment, a proximal end of the wetting fluid compartment encircles a distal part of the catheter shaft and distal end of the wetting fluid compartment encircles a proximal end of the connector, such as in the illustrative examples of Figs. 1 and 3.

The wetting fluid compartment may thus have a first, proximal opening and a second, distal opening. The openings are preferably closed by the non-insertable part of the medical device, ensuring that the wetting fluid does not escape from the wetting fluid compartment prior to withdrawal of the medical device through the wetting fluid compartment. Such closing can e.g. be obtained by a friction fit, whereby the inner diameters of the openings are essentially the same as the corresponding outer diameters of the non-insertable part at the positions where the openings are arranged.

In one embodiment, at least one breakable seal (not shown) is further provided between the wetting fluid compartment and the elongate shaft, and preferably two breakable seals, provided on opposite sides of the wetting fluid compartment. Thus, the seals are hereby preferably arranged between the inner surface of the openings and the corresponding outer surfaces of the non-insertable part. Such seals may e.g. be formed by an adhesive, rubber or the like.

Regardless of whether seals are used or not, the diameter of the proximal opening of the wetting fluid compartment is preferably essentially the same as the outer diameter of the elongate shaft, or only slightly greater.

At least the proximal opening of the wetting fluid compartment is preferably circular, and most preferably both the proximal and the distal openings are circular.

In an embodiment, the distal opening of the wetting fluid compartment has a greater diameter than the diameter of the proximal opening, such as illustrated in the embodiments of Figs. 1 and 3. Here, the proximal opening of the wetting fluid compartment 4, 4' has essentially the same inner diameter as the outer diameter of the catheter shaft, which it overlies. The distal opening of the wetting fluid compartment 4, 4' overlies a part 24 of the non-insertable section 22, which has an outer diameter which is greater than the outer diameter of the catheter shaft. The distal opening of the wetting fluid compartment here has essentially the same inner diameter as the outer diameter of the part 24. Hereby, a slight distance may be formed between the elongate shaft and the distal opening when the elongate shaft is pulled out through the wetting fluid compartment.

Upon use, the medical device will be pulled in relation to the wetting fluid compartment, thereby breaking the seals, if such seals are present. However, since at least the proximal opening of the wetting fluid compartment is still essentially closed by the elongate shaft, the aqueous fluid will not pour out from the wetting fluid compartment during the withdrawal of the elongate shaft through the wetting fluid compartment. Due to capillary forces, the wetting fluid will instead be maintained in the compartment until the proximal tip of the elongate shaft has reached and passed through the proximal opening of the wetting fluid compartment. Any fluid remaining in the wetting fluid compartment will pour out only after complete removal of the elongate shaft, thus ensuring a complete wetting of the hydrophilic coating/surface.

To ensure that adequate wetting is obtained in the relatively short time during which each part of the hydrophilic coating/surface is exposed to the aqueous fluid, at least one of the aqueous fluid and the hydrophilic coating/surface includes at least one surfactant. In a preferred embodiment, the aqueous fluid includes the at least one surfactant. However, additionally or alternatively, the at least one surfactant may be included in the hydrophilic coating/surface.

The relative amount of surfactant of the fluid phase (the wetting fluid or the fluid in the wetted coating/surface) may be in the vicinity of the Critical Micelle Concentration for the surfactant(s).

The total concentration of surfactant(s) may be in the range of 0.001-1 wt%, of the fluid phase (the wetting fluid or the fluid in the wetted coating/surface) and preferably in the range 0.005-0.5 wt%, and more preferably in the range 0.01-0.1 wt%, and most preferably in the range 0.01-0.08 wt%.

Expressed in Molar (M), the total concentration of surfactant(s) may be in the range of 0.001-1 mM, of the liquid phase (the wetting fluid or the fluid in the wetted coating/surface) and preferably in the range 0.002-0.7 mM, and more preferably in the range 0.003-0.6 mM.

The surfactant is preferably water soluble, and may be non-ionic surfactant. However, other surfactants may also be used, such as zwitterionic, anionic or cationic. Further, the surfactant preferably has relatively large chains/molecules, which further reduces interaction with human tissue.

The aqueous fluid may further include a polyol, such as glycerol, and/or a low molecular weight hydrophilic polymer, such as PVP. In one embodiment, the aqueous fluid includes a relative amount of polyol/hydrophilic polymer in the range of 0.01-20 wt%, and preferably 0.05-15 wt%, and more preferably 0.1-10 wt%, and most preferably 1-8 wt%, such as about 5 wt%. The polyol/hydrophilic polymer provides protection for the wetting fluid, and in particular preserves the function of the surfactants, during radiation sterilization. The polyol/hydrophilic polymer reduces the crosslinking/reaction of the surfactant which may otherwise occur during radiation sterilization.

The aqueous fluid may further comprise an osmolality increasing compound, such as sodium chloride.

The aqueous fluid may also comprise a pH buffer, to ensure that the pH of the fluid is maintained in a preferred range, such as in the range of 4-8. The pH buffer may be citric acid, both other buffers may also be used, such as acetic acid, phosphate buffer, carboxylic acids, amino acids, aminosulphonic acids and inorganic acids.

The aqueous fluid preferably comprises at least 80 wt% water, and more preferably at least 90 wt%, and most preferably at least 95 wt%.

In embodiments where the aqueous fluid includes a low molecular weight polyol and/or hydrophilic polymer, the fluid preferably comprises 80-99 wt% water and 1-10 wt% polyol/hydrophilic polymer.

Preferably, the fluid contains primarily water, and optionally a polyol, such as glycerol, and/or a low molecular weight hydrophilic polymer, such as PVP. The total concentration of additives in addition to water and polyol/hydrophilic polymer, such as the surfactant, preferably is equal to or less than 5 wt%, and more preferably less than 3 wt%, and even more preferably less than 1 wt%, and most preferably less than 0.5 wt%. Apart from the surfactant, such additional additives may include one or more of an osmolality increasing agent, a pH buffer, an antibacterial agent, fragrances, a pharmaceutical active substance, or the like.

In the present solution, it is possible to use a very small volume of the aqueous fluid, since it is continuously applied to the hydrophilic coating/surface only where it is needed. Thus, it in principle suffice with a volume corresponding to the volume which is absorbed by the hydrophilic coating/surface for swelling of the same. In one embodiment, the wetting fluid compartment may comprise an amount of the aqueous fluid in the range of 0.5-5 ml, and preferably in the range of 1-4 ml. Such a small amount of wetting fluid will be sufficient for complete wetting of a hydrophilic coating/surface of a male urinary catheter.

The wetting fluid compartment is preferably impermeable to the wetting fluid, and preferably made of a gas impermeable material.

### Experimental results

The effect of using a surfactant in the wetting fluid has been demonstrated in a number of experiments. For the tests, four solutions were prepared as comparative examples (C1a-C1c and C2), and several solutions as examples of the invention (E1-E10a). The following different wetting liquids were tested:
- C1: Plain water (as a comparative example)
- C1a: Water with 7 wt% glycerol (as another comparative example)
- C1b: Water with 7.5 wt% PVP K12 (as another comparative example)
- C2: Water with 2.5 wt% NaCl (as another comparative example)
- E1: Water with 0.077 wt% (0.5878 mM) Tween 80
- E1a: Water with 0.076 wt% (0.5802 mM) Tween 80 and 7 wt% glycerol
- E2: Water with 0.019 (0.0152 mM) wt% Kolliphor p407
- E2a: Water with 0.019 wt% (0.0152 mM) Kolliphor p407 and 7 wt% glycerol
- E3b: Water with 0.011 wt% (0.0088 mM) Kolliphor p407 and 7.5 wt% PVP K12
- E4a: Water with 0.042 wt% (0.0420 mM) HPMC (Mn ^{∼}10,000 / 6 cP) and 7 wt% glycerol
- E5: Water with 0.010 wt% (0.0076 mM) Kolliphor p407
- E6: Water with 0.005 wt% (0.0038 mM) Kolliphor p407
- E7: Water with 0.019 wt% (0.1667 mM) Brij 35
- E7a: Water with 0.019 wt% (0.1667 mM) Brij 35 and 7 wt% glycerol
- E8: Water with 0.019 wt% TEGO Betain F50
- E8a: Water with 0.019 wt% TEGO Betain F50 and 7 wt% glycerol
- E9a: Water with 0.019 wt% Kolliphor p188 and 7 wt% glycerol
- E10a: Water with 0.018 wt% HPMC HME 4M and 7 wt% glycerol

For all the tests, LoFric^{®} catheters from Wellspect have been used. The catheters were CH 14, with a length of 40 cm. Such catheters are commercially available, and have a hydrophilic coating comprising PVP, which obtains a low friction when wetted.

In a first line of experiments, the friction and wetting were evaluated manually. For these tests, a container comprising a liquid solution was arranged over the non-insertable section of the coated dry catheter, and an amount of wetting liquid was added to the container. The wetting liquid was maintained in the container when the catheter was manually drawn vertically up through the container at a high speed (about 30 cm/s) or a low speed (about 3 cm/s). The height of the liquid in the container that the catheters were drawn through, i.e. the liquid pillar height extending around the catheter, was either 2 cm or 7.5 cm. All the high speed tests were made with a 2 cm liquid height.

After having been pulled out through the container, the wetted catheters were hung vertically for a dry-out time of 90 s.

Thereafter, the slipperiness of the wetted hydrophilic coatings were assessed by pulling fingers along the shaft, and in particular to identify unevenness, such as presence of dry areas and areas with higher friction.

The assessed catheters were graded to be in one of the categories:
- 5: Very good
- 4: Good
- 3: OK
- 2: Several dry spots
- 1: Several large dry spots
- 0: No slipperiness

For some of the tests, the wetting liquid was radiation sterilized prior to wetting, and for others, the wetting liquid was not radiation sterilized.

The results of the manual assessment are presented in Table 1 below.

**Table 1: Manual assessment of slipperiness**

| Wetting solution | Speed | Liquid height | Manual assessment Non-sterile | Manual assessment Sterile |
|---|---|---|---|---|
| C1 | High | 2cm | 1 | 0 |
| C1a | High | 2cm | 1 | 1 |
| C1b | High | 2cm | 2 | 0 |
| C2 | High | 2cm | N/A | 1 |
| E1 | High | 2cm | 5 | 1 |
| E1a | High | 2cm | 4-5 | 5 |
| E2 | High | 2cm | 5 | 1 |
| E2a | High | 2cm | 5 | 5 |
| E3b | High | 2cm | 5 | 4-5 |
| E4a | High | 2cm | 4-5 | 4-5 |
| E2 | Low | 2cm | 3 | N/A |
| E2 | Low | 7.5cm | 4-5 | N/A |
| E5 | High | 2cm | 4 | N/A |
| E6 | High | 2cm | 2 | N/A |
| E6 | Low | 2cm | 1 | N/A |
| E6 | Low | 7.5cm | 3 | N/A |
| E7 | High | 2cm | 5 | 2 |
| E7b | High | 2cm | 5 | 5 |
| E8 | High | 2cm | N/A | 2 |
| E8a | High | 2cm | N/A | 5 |
| E8a | Low | 2cm | N/A | 3 |
| E9a | High | 2cm | N/A | 5 |
| E10a | High | 2cm | N/A | 5 |

From this it can be deduced that all the comparative examples perform very poorly, and do not become adequately wetted by this wetting process.

All the inventive examples perform adequately, and are sufficiently wetted by the wetting process. It can be noted that too low addition of surfactant can lead to a somewhat poorer wetting - E2 at high speed performs better than E5, and much better than E6. It can also be noted that low speed does not make the performance better, but rather the opposite - E2 and E8a performs better at high speed. However, this can at least to some extent be compensated by having a greater liquid height - seen in E2 and E6.

It can also be noted that if the wetting liquid is sterilized by gamma or beta radiation, it may perform somewhat poorer after sterilization. See e.g. E1, E2 and E7. However, this can be effectively remedied by adding a polyol, such as glycerol, to the wetting liquid. See e.g. E1a, E2a, E4a, E8a, E9a and E10a. Addition of PVP in the wetting liquid also seems to have a similar protective effect - see e.g. E3b and E7b.

In another line of experiments, the water retention, or fluid dry out time, was tested. For these tests, the catheters were dipped very quickly, in 1-2 seconds, in a wetting liquid. Thereafter, the water retention/fluid dry out time of the catheters was determined by determining the water/fluid content (mg/cm²) in the hydrophilic coating after 1 and 6 minutes drying in air. The water/fluid content was determined by weighing the catheters before wetting, to obtain a reference weight, and to weigh the catheters 1 and 6 minutes after wetting, and subtracting the reference weight from this measurement. The area of coating is measured. The obtained weight/cm² difference is a measure on the water/fluid content being held by the hydrophilic coating at the time of measurement.

A number of different wetting liquids were tested. Each of the wetting liquids was tested multiple times, and the presented results are the average values. The results are presented in Table 2 below.

**Table 2: Water retention/fluid dry out time in coating after 1 and 6 minutes**

| Wetting solution | Radiation sterilized | Water retention after 1 minute [mg/cm2] | Water retention after 6 minutes [mg/cm2] |
|---|---|---|---|
| C1 | No | 8.39 | 6.53 |
| C1a | No | 7.96 | 6.40 |
| C1a | Yes | 8.35 | 6.61 |
| C1b | No | 7.05 | 5.21 |
| E1 | No | 10.74 | 8.75 |
| E1 | Yes | 9.04 | 6.94 |
| E1a | Yes | 10.48 | 8.60 |
| E2 | No | 10.18 | 8.19 |
| E2 | Yes | 8.65 | 6.80 |
| E2a | Yes | 10.73 | 8.90 |
| E3b | Yes | 9.21 | 7.39 |
| E5 | No | 10.79 | 8.70 |
| E6 | No | 10.41 | 8.56 |
| E7 | No | 10.93 | 8.77 |

From this it can be deduced that all the inventive examples perform better than the comparative examples, both after 1 minute and 6 minutes. It can also be noted that when radiation sterilization has been used, the wetting liquids having an addition of glycerol and/or PVP perform better.

The slipperiness of the catheters was, as discussed in the foregoing, evaluated by manual assessment. However, in order to confirm this, some of the samples were evaluated using a Harland FTS Friction Tester, available from Harland Medical Systems, with a clamp force of 100 g. To this end, the catheters were dipped in a wetting liquid, and immediately pulled out by the friction tester during continuous measurement of the friction. The first part of the catheters pulled out and measured were disregarded, since these parts were not wetted. The measured parts were wetted about 1-2 seconds.

The results of this are presented in the Table 3 below.

**Table 3: Coefficient of friction results**

| Wetting solution | Friction coefficient |
|---|---|
| C1 | 1.72 |
| E2 | 0.33 |
| E7 | 0.65 |

These results confirm the above-discussed findings, and both E2 and E7 have much lower friction than the comparative example C1.

In further experiments it has been found that the pH of the wetting liquid is generally not affected by the addition of the surfactant.

### Concluding remarks

Specific embodiments of the invention have now been described. However, several alternatives are possible, as would be apparent for someone skilled in the art. For example, many different surfactants can be used, and in various concentrations. Further, other additives, such as polyol and/or a low molecular weight hydrophilic polymer, may be beneficial in certain circumstances, but may not be necessary in other.

The arrangement of the wetting liquid compartment to enable withdrawal of the catheter shaft through the compartment may be accomplished in many different ways. Further, the teachings of this invention are not only of advantage for urinary catheters, but also for many other types of hydrophilic catheters and hydrophilic medical devices.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. A medical device assembly comprising:
a medical device comprising an insertable part and a non-insertable part, wherein the insertable part comprises an elongate shaft provided with a hydrophilic coating or surface;
a wetting fluid compartment arranged to encircle at least a part of the medical device, and preferably at least a part of the non-insertable part; and
an aqueous fluid arranged in the wetting fluid compartment, wherein at least one of the aqueous fluid and the hydrophilic coating or surface comprises at least one surfactant;
wherein the hydrophilic coating or surface is maintained essentially separated from the aqueous fluid, and arranged to become wetted by movement of the elongate shaft through the wetting fluid compartment.

2. The medical device assembly of claim 1, wherein a total concentration of surfactant(s) is in the range of 0.001-1 wt%, of the liquid phase (the wetting fluid or the fluid in the wetted coating/surface) and preferably in the range 0.005-0.5 wt%, and more preferably in the range 0.01-0.1 wt%, and most preferably in the range 0.01-0.08 wt%.

3. The medical device assembly of claim 1 or 2, wherein a total concentration of surfactant(s) is in the range of 0.001-1 mM, of the liquid phase (the wetting fluid or the fluid in the wetted coating/surface), and preferably in the range 0.002-0.7 mM, and more preferably in the range 0.003-0.6 mM.

4. The medical device assembly of any one of the preceding claims, wherein the aqueous fluid further includes a polyol, such as glycerol, and/or a low molecular weight hydrophilic polymer, such as PVP.

5. The medical device assembly of claim 4, wherein the aqueous fluid includes a relative amount of polyol and/or low molecular weight hydrophilic polymer in the range of 0.01-20 wt%, and preferably 0.05-15 wt%, and more preferably 0.1-10 wt%, and most preferably 1-8 wt%.

6. The medical device assembly of any one of the preceding claims, wherein the surfactant is a non-ionic surfactant.

7. The medical device assembly of any one of the preceding claims, wherein the medical device is a catheter, and preferably a urinary catheter.

8. The medical device assembly of any one of the preceding claims, further comprising a package accommodating the wetting fluid compartment and at least the insertable part of the medical device.

9. The medical device assembly of any one of the preceding claims, wherein the wetting fluid compartment is fixedly arranged in the package, wherein extraction of the medical device from the package simultaneously provides a movement of the elongate shaft through the wetting fluid compartment, thereby wetting the hydrophilic coating or surface.

10. The medical device assembly of any one of the claims 1-8, wherein the wetting fluid compartment is releasable from the package.

11. The medical device assembly of any one of the preceding claims, wherein the medical device is a catheter comprising at least one drainage opening arranged in a sidewall of the elongate shaft, wherein the wetting fluid compartment has a height in a longitudinal direction of the catheter exceeding a length of the drainage opening in the longitudinal direction of the catheter.

12. The medical device assembly of any one of the preceding claims wherein the medical device is a catheter comprising at least two drainage openings arranged in a sidewall of the elongate shaft, wherein the wetting fluid compartment has a height in a longitudinal direction of the catheter exceeding a length between the ends of the drainage openings being farthest apart in the longitudinal direction of the catheter.

13. The medical device assembly of any one of the preceding claims, wherein at least one breakable seal is provided between the wetting fluid compartment and the elongate shaft, and preferably two breakable seals, provided on opposite sides of the wetting fluid compartment.

14. The medical device assembly of any one of the preceding claims, wherein the wetting fluid compartment comprises an amount of the aqueous fluid in the range of 0.5-5 ml, and preferably in the range of 1-4 ml.

15. The medical device assembly of any one of the preceding claims, wherein the height of the level of wetting fluid within the wetting liquid compartment is at least 2 cm, and preferably at least 4 cm, and most preferably at least 6 cm.

16. A method for wetting a hydrophilic medical device comprising:
providing a medical device comprising an insertable part and a non-insertable part, wherein the insertable part comprises an elongate shaft provided with a hydrophilic coating or surface;
providing a wetting fluid compartment containing an aqueous fluid around at least a part of the non-insertable part, wherein at least one of the aqueous fluid and the hydrophilic coating or surface comprises at least one surfactant;
moving the elongate shaft in relation to the wetting fluid compartment, thereby wetting the hydrophilic coating/surface.
